# EUROPEAN PATENT SPECIFICATION

(11) **EP 3 775 125 B1**
(45) Date of publication and mention of the grant of the patent: **28.09.2022**
(21) Application number: 19716174.8
(22) Date of filing: 10.04.2019
(51) Int. Cl.: C11D 1/825, C11D 1/83, C11D 1/94, A61K 8/60, A61Q 19/10, C11D 1/66

(54) **USE OF A RHAMNOLIPID BIOSURFACTANT TO INCREASE THE VISCOSITY OF A FLUID CLEANING COMPOSITION**
VERWENDUNG EINES RHAMNOLIPID-BIOTENSIDS ZUR ERHÖHUNG DER VISKOSITÄT EINER FLÜSSIGEN REINIGUNGSZUSAMMENSETZUNG
UTILISATION D'UN BIOSURFACTANT RHAMNOLIPIDE POUR AUGMENTER LA VISCOSITÉ D'UNE COMPOSITION DE NETTOYAGE FLUIDE

(30) Priority: 17.05.2018 EP 18172982
(43) Date of publication of application: 17.02.2021
(73) Proprietor: Unilever IP Holdings B.V., 3013 AL Rotterdam (NL); Unilever Global IP Limited, Wirral, Merseyside CH62 4ZD (GB)
(72) Inventor: JONES, Clare, Anne, Wirral Merseyside CH63 3JW (GB); STEVENSON, Paul, Simon, Wirral Merseyside CH63 3JW (GB)
(74) Representative: McHugh, Paul Edward
(86) International application number: PCT/EP2019/059167
(87) International publication number: WO 2019/219303

(56) References cited:
- EP-A1- 2 786 742
- WO-A1-2018/065314
- US-A1- 2014 349 902
- US-A1- 2016 045 424
- US-A1- 2017 335 238
- US-A1- 2018 016 525
- US-A1- 2018 044 614

## Description

### Field of Invention

The present invention relates to the use of a rhamnolipid biosurfactant to increase the viscosity of a fluid cleaning composition.

### Background of the Invention

The rheology of many fluid cleaning compositions could be improved in the eyes of the consumer. Consumers dislike fluid cleaning compositions that have a too low viscosity, as there can be a perception that these compositions are not thick enough to provide adequate cleaning. Thus, there is a problem of how to improve the viscosity of fluid cleaning compositions.

US 2016/0045424 A1 relates to a method of thickening a cosmetic formulation comprising introducing at least one specific water-soluble carbomer copolymer to a cosmetic formulation comprising at least one surfactant selected from the group consisting of sulphosuccinates and biosurfactants.

US 2018/0044614 A1 relates to a viscous perfumed cleaning fluid for a cleaning a substrate comprising: (a) a surfactant combination comprising at least one surfactant, and a glycolipid biosurfactant which is present at a level in the range 10-95 wt % of the total surfactant in said surfactant system, (b) one or more viscosity modifiers, (c) ethoxylated polyethylene imine (EPEI), and (d) a volatile benefit agent; wherein the composition is adjusted to a pH of at least 5, preferably at least 6, and more preferably at least 7.

### Summary of the Invention

We have found that by adding rhamnolipid biosurfactant to a fluid cleaning composition, and by having a pH in the range in the range of from 4 to 5.5, improved rheology in terms of higher viscosity can be achieved.

The invention provides the use of a rhamnolipid biosurfactant to increase the viscosity of a fluid cleaning composition at a pH of 4 to 5.5, preferably from 4.5 to 5.5.

Preferably the rhamnolipid biosurfactant comprises at least 70 wt.% di-rhamnolipid, preferably at least 80 wt.% di-rhamnolipid, preferably of formula: Rha2C₈-₁₂C₈₋₁₂, where the alkyl chains may be saturated or unsaturated.

### Detailed Description of the Invention

The fluids cleaning compositions are in particular, but not exclusively, for use in aqueous based treatments such as personal bathing (e.g. a personal wash or shampoo composition), washing of fabrics (e.g. a laundry liquid composition) and dishes (e.g. a hand dishwash liquid composition).

Preferably the fluid cleaning compositions are home care compositions, for example useful for the washing of fabrics (e.g. a laundry liquid composition) and dishes (e.g. a hand dishwash liquid composition).

Where viscosities are measured herein, unless otherwise stated, they are measured on an Anton Paar ASC Rheometer at 25°C.

Example viscosity of the composition is in the range 250 - 1000 Cps for laundry liquids and 400 - 4000 Cps for hand dishwash liquid formulations. Preferably the pour viscosity of the composition is in the range 300 - 650 Cps for laundry liquid and 800 - 3500 Cps for hand dish wash formulations.

The fluid cleaning composition comprises from 5 to 70 wt.% of a surfactant system.

Preferably the fluid cleaning composition comprises from 5 to 60 wt.%, more preferably from 5 to 50 wt.%, even more preferably from 7.5 to 30 wt.%, most preferably from 7.5 to 25 wt.%, for example from 8 to 25 wt.%, or even from 8 to 20 wt.%, of a surfactant system.

The surfactant system comprises at least one anionic or nonionic surfactant and a rhamnolipid biosurfactant which is present at a level in the range of from 1 to 95 wt.% of the total surfactant in said surfactant system; wherein the composition comprises from 0.5 to 10 wt.% of an amphoteric/zwitterionic surfactant, said surfactant being counted as part of the surfactant system; and, wherein the composition comprises a non-ionic surfactant, wherein the non-ionic surfactant is an alcohol ethoxylate.

Preferably the rhamnolipid is present at a level in the range of from 1 to 50 wt.%, of the total surfactant in said surfactant system, more preferably from 2.5 to 50 wt.%, even more preferably from 5 to 25 wt.%, for example from 7.5 to 25 wt.% of the total surfactant in said surfactant system.

The fluid cleaning compositions comprise water. Water is usually the balancing agent in the formulation, and may make up all or the bulk of the non-surfactant wt.% in the composition. Typical water inclusion levels may be from 50 to 90 wt.%, preferably from 60 to 90 wt.%, more preferably from 65 to 88 wt.%.

### Rhamnolipid

The composition comprises a rhamnolipid biosurfactant.

Mono-rhamnolipids have a single rhamnose sugar ring.

Di-rhamnolipids have two rhamnose sugar rings.

In the case of rhamnolipids, throughout this patent specification, the prefixes mono- and di- are used to indicate respectively to indicate mono-rhamnolipids (having a single rhamnose sugar ring) and di-rhamnolipids (having two rhamnose sugar rings) respectively. If abbreviations are used R1 is mono-rhamnolipid and R2 is di-rhamnolipid.

The mono-rhamnolipid may be L-rhamnosyl-β-hydroxydecanoyl-β-hydroxydecanoate (RhaC₁₀C₁₀ with a formula of C₂₆H₄₈O₉) produced by *P. aeruginosa.*

Atypical di-rhamnolipid is L-rhamnosyl-L-rhamnosyl-β-hydroxydecanoyl-β-hydroxydecanoate (Rha2C₁₀C₁₀ with a formula of C₃₂H₅₈O₁₃).

In practice a variety of other minor components with different alkyl chain length combinations, depending upon carbon source and bacterial strain, exist in combination with the above more common rhamnolipids. The ratio of mono-rhamnolipid and di-rhamnolipid may be controlled by the production method. Some bacteria only produce mono-rhamnolipid, see US5767090: Example 1, some enzymes can convert mono-rhamnolipid to di-rhamnolipid.

The following rhamnolipids are sources of mono- and di- rhamnolipids encompassed within the invention (C12:1, C14:1 indicates fatty acyl chains with double bonds):
- Rhamnolipids produced by *P. aeruginosa* (mono-rhamnolipids):
   Rha-C₈-C₁₀, Rha-C₁₀-C₈, Rha-C₁₀-C₁₀, Rha-C₁₀-C₁₂, Rha-C₁₀-C_{12:1}, Rha-C₁₂-C₁₀, Rha-C_{12:1}C₁₀
- Rhamnolipids produced by *P. chlororaphis* (mono-rhamnolipids only):
   Rha-C₁₀-C₈, Rha-C₁₀-C₁₀, Rha-C₁₂-C₁₀, Rha-C_{12:1}-C₁₀, Rha-C₁₂-C₁₂, Rha-C_{12:1}C₁₂, Rha-C₁₄-C₁₀, Rha-C_{14:1}-C_{10.}
- Mono-rhamnolipids may also be produced from *P.putida* by introduction of genes rhIA and rhIB from *Psuedomonas* aeruginosa [Cha et al. in Bioresour Technol. 2008. 99(7):2192-9 ]
- Rhamnolipids produced by *P. aeruginosa* (di-rhamnolipids):
   Rha-Rha-C₈-C₁₀, Rha-Rha-C₈-C_{12:1}, Rha-Rha-C₁₀-C₈, Rha-Rha-C₁₀-C₁₀, Rha-Rha-C₁₀-C_{12:1}, Rha-Rha-C₁₀-C₁₂, Rha-Rha-C₁₂-C₁₀, Rha-Rha-C_{12:1}-C₁₂, Rha-Rha-C₁₀-C_{14:1}
- Rhamnolipids produced by *Burkholdera pseudomallei* (di-rhamnolipids only):
   Rha-Rha-C₁₄-C₁₄.
- Rhamnolipids produced by *Burkholdera (Pseudomonas) plantarii* (di-rhamnolipids only):
   Rha-Rha-C₁₄-C_{14.}
- Rhamnolipids produced by *P. aeruginosa* which are initially unidentified as either mono- or di-rhamnolipids:
   C₈-C₈, C₈-C₁₀, C₁₀-C₈, C₈-C_{12:1}, C_{12:1}-C₈, C₁₀-C₁₀, C₁₂-C₁₀, C_{12:1}-C₁₀, C₁₂-C₁₂, C_{12:1}-C₁₂, C₁₄-C₁₀, C_{14:1}-C₁₀, C₁₄-C₁₄.

Preferably, the rhamnolipid comprises at least 50 wt.% di-rhamnolipid, more preferably at least 60 wt.% di-rhamnolipid, even more preferably 70 wt.% di-rhamnolipid, most preferably at least 80 wt.% di-rhamnolipid.

Preferably the rhamnolipid is a di-rhamnolipid of formula: Rha2C₈-₁₂C₈₋₁₂. The preferred alkyl chain length is from C₈ to C₁₂, the alkyl chain may be saturated or unsaturated.

### Surfactant

The fluid cleaning composition comprises at least one anionic and/or non-ionic surfactant. This means that the composition may comprises a single anionic surfactant, or a mixture of anionic surfactants, or a single nonionic surfactant, or a mixture of nonionic surfactant, or a mixture of one or more anionic surfactants with one or more nonionic surfactants.

Suitable nonionic and anionic surfactants may be chosen from the surfactants described "Surface Active Agents" Vol. 1, by Schwartz & Perry, Interscience 1949, Vol. 2 by Schwartz, Perry & Berch, Interscience 1958, in the current edition of "McCutcheon's Emulsifiers and Detergents" published by Manufacturing Confectioners Company or in "Tenside-Taschenbuch", H. Stache, 2nd Edn., Carl Hauser Verlag, 1981 or in Anionic Surfactants: Organic Chemistry edited by Helmut W. Stache (Marcel Dekker 1996).

Suitable anionic detergent compounds which may be used are usually water-soluble alkali metal salts of organic sulphates and sulphonates having alkyl radicals containing from about 8 to about 22 carbon atoms, the term alkyl being used to include the alkyl portion of higher alkyl radicals.

Examples of suitable synthetic anionic detergent compounds are sodium and potassium alkyl sulphates, especially those obtained by sulphating higher C₈ to C₁₈ alcohols, produced for example from tallow or coconut oil, Alkyl ether carboxylic acids; sodium and potassium alkyl C₉ to C₂₀ benzene sulphonates, particularly sodium linear secondary alkyl C₁₀ to C₁₅ benzene sulphonates; and sodium alkyl glyceryl ether sulphates, especially those ethers of the higher alcohols derived from tallow or coconut oil and synthetic alcohols derived from petroleum.

The anionic surfactant is preferably selected from: linear alkyl benzene sulphonate; alkyl sulphates; alkyl ether sulphates; alkyl ether carboxylates, and mixtures thereof.

More preferred anionic surfactants are selected from: linear alkyl benzene sulphonate; alkyl sulphates; alkyl ether sulphates and mixtures thereof. Preferably the alkyl ether sulphate is a C₁₂-C₁₄ n-alkyl ether sulphate with an average of 1 to 3EO (ethoxylate) units. Sodium lauryl ether sulphate is particularly preferred (SLES). Preferably the linear alkyl benzene sulphonate is a sodium C₁₁ to C₁₅ alkyl benzene sulphonates. Preferably the alkyl sulphate is a linear or branched sodium C₁₂ to C₁₈ alkyl sulphates. Sodium dodecyl sulphate is particularly preferred, (SDS, also known as primary alkyl sulphate).

Preferably the anionic surfactant comprises linear alkyl benzene sulphonates and/or alkyl ether sulphates.

Preferably two or more anionic surfactants are present, for example linear alkyl benzene sulphonate together with an alkyl ether sulphate.

Suitable nonionic detergent compounds which may be used include, in particular, the reaction products of compounds having an aliphatic hydrophobic group and a reactive hydrogen atom, for example, aliphatic alcohols, acids or amides, especially ethylene oxide either alone or with propylene oxide. Specific nonionic detergent compounds are the condensation products of aliphatic C₈ to C₁₈ primary or secondary linear or branched alcohols with ethylene oxide.

The composition comprises non-ionic surfactant, wherein the non-ionic surfactant is an alcohol ethoxylate, more preferably a C₁₀-C₁₈ alcohol ethoxylate having an average of 3-10 moles of ethylene oxide, most preferably a C₁₂-C₁₅ alcohol ethoxylate having an average of 5-9 moles of ethylene oxide.

Preferably the surfactants used are saturated.

### Zwitterionic/Amphoteric surfactant

The surfactant combination comprises from 0.5 to 10 wt.% of one or more amphoteric/zwitteronic surfactants.

Preferably the composition comprises from 0.75 to 5 wt.%, most preferably from 1 to 4 wt.% of an amphoteric/zwitterionic surfactant, preferably a betaine surfactant, said surfactant being counted as part of the surfactant system.

Preferred amphoteric/zwitteronic surfactants are betaine surfactants. A preferred betaine surfactant is cocoamidopropyl betaine.

A preferred fluid cleaning composition comprises:
a) from 5 to 50 wt.% of a surfactant system comprising:
   i) at least one anionic and/or nonionic surfactant; and
   ii) a rhamnolipid biosurfactant which is present at a level in the range of from 1 to 95 wt.%, preferably from 1 to 50 wt.%, more preferably from 2.5 to 50 wt.%, most preferably from 5 to 25 wt.% of the total surfactant in said surfactant system; and
   iii) from 0.5 to 10 wt.% of a betaine surfactant;
b) water;
c) from 0.1 to 2 wt.% of a perfume
wherein the composition has a pH of from 4 to 5.5;
wherein the composition comprises from 0.5 to 10 wt.%, preferably from 0.75 to 5 wt.%, most preferably from 1 to 4 wt.% of an amphoteric/zwitterionic surfactant, said surfactant being counted as part of the surfactant system;
wherein the composition comprises a non-ionic surfactant, wherein the non-ionic surfactant is an alcohol ethoxylate, preferably a C₁₀-C₁₈ alcohol ethoxylate having an average of 3-10 moles of ethylene oxide, more preferably a C₁₂-C₁₅ alcohol ethoxylate having an average of 5-9 moles of ethylene oxide; and,
wherein the rhamnolipid biosurfactant comprises at least 70 wt.% di-rhamnolipid, preferably at least 80 wt.% di-rhamnolipid, preferably of formula: Rha2C₈-₁₂C₈₋₁₂, where the alkyl chains may be saturated or unsaturated.

### pH

The fluid cleaning compositions have a pH of from 4 to 5.5, preferably from 4.5 to 5.5.

### Perfume

The composition preferably comprises a perfume. The perfume is preferably present in the range from 0.001 to 3 wt.%, more preferably 0.05 to 0.5 wt.%, even more preferably from 0.1 to 2 wt.%, most preferably 0.1 to 1 wt.%.

Many suitable examples of perfumes are provided in the CTFA (Cosmetic, Toiletry and Fragrance Association) 1992 International Buyers Guide, published by CFTA Publications and OPD 1993 Chemicals Buyers Directory 80th Annual Edition, published by Schnell Publishing Co.

The perfume can be provided as a free oil, or may in encapsulated form.

### Other ingredients

Preferably the composition comprises an ionic salt. The salt preferably comprises any organic or inorganic cation, including without limitation cations of alkali metals Cs, Na, K, Ca, Mg etc., with anions including halide anions, more preferably Cl. Other preferred salts comprise organic cations e.g. amides (- ⁺NH-R ) or ammonium cations or substituted forms thereof e.g. triethylammonium. Anions for organic cations may comprise any alkyl, aryl, arylalkyl moiety which may be short, medium, long, branched, cyclic or linear.

Preferably the composition comprises from 0.01 - 5wt.% by weight of the salt. In the case of NaCl, preferably the level is in the range 0.5 - 2 wt.%

Fluid cleaning compositions may, depending on their end use further comprise any of the following as a single ingredient, or a mixture thereof: polymers, polyester substantive soil release polymers, hydrotropes, opacifiers, preservatives, colorants (e.g. dyes and pigments), enzymes (for example proteases, alpha-amylases, cellulases, lipases, peroxidases/oxidases, pectate lyases, and mannanases, or mixtures thereof), further surfactants such as cationic surfactants, softeners, polymers for anti redeposition of soil, bleach, bleach activators and bleach catalysts, antioxidants, pH control agents and buffers.

Where alkyl groups are sufficiently long to form branched or cyclic chains, the alkyl groups encompass branched, cyclic and linear alkyl chains. The alkyl groups are preferably linear or branched, most preferably linear.

The indefinite article "a" or "an" and its corresponding definite article "the" as used herein means at least one, or one or more, unless specified otherwise.

### Experimental

The examples below are intended to illustrate the invention in detail without, however, limiting it thereto. Examples denoted by a letter are comparative, examples denoted by a number are according to the invention.

### Example 1

This example shows the effect of rhamnolipid on the viscosity of a hand dishwash formulation at low pH.

**Table 1: Hand dishwash formulation**

| **Ingredient** | **A (wt.%)** | **1 (wt.%)** |
|---|---|---|
| Demin water | 81.59 | 81.59 |
| LAS (acid?) | 5.2 | 4.68 |
| NaOH (47% solution) | 0.62 | 0.62 |
| Citric Acid (50% solution) | 0.07 | 0.07 |
| Microcare IT | 0.0009 | 0.0009 |
| Glydant | 0.22 | 0.22 |
| Tetrasodium EDTA | 0.10 | 0.10 |
| R2 Rhamnolipid | - | 1.45 |
| Texapon N701 SLES 1EO | 9.3 | 8.37 |
| Magnesium sulphate heptahydrate | 2.70 | 2.70 |
| Perfume | 0.20 | 0.20 |
| Colourant | 0.0002 | 0.0002 |

The pH of the base was adjusted with small amounts of diluted citric acid and sodium hydroxide solutions, and the viscosity was measured using the Anton Paar ASC rheometer-using a Bob set-up and reporting the viscosity measured at a shear rate of 23s⁻¹ and shown in tables 2 and 3.

**Table 2 The rheology (viscosity) of the HDW formulation [formulation A (Control)] at a shear rate of 23s⁻¹ when no Rhamnolipid is present**

| **Formulation A (0% Rhamnolipid)** | |
|---|---|
| **pH** | **Viscosity (Cps)** |
| 5.00 | 1370 |
| 5.08 | 1370 |
| 5.43 | 1350 |
| 5.46 | 1300 |
| 5.54 | 1300 |
| 5.94 | 1400 |
| 6.25 | 1320 |

**Table 3 The rheology (viscosity) of the HDW formulation [formulation 1 (Invention)] at a shear rate of 23s⁻¹ when 10% of the total active system has been replaced by Rhamnolipid**

| **Formulation 1 (10% Rhamnolipid replacement)** | |
|---|---|
| **pH** | **Viscosity (Cps)** |
| 6.54 | 204 |
| 6.00 | 972 |
| 5.52 | 1720 |
| 4.73 | 2460 |
| 4.50 | 2640 |

This data shows the improvement in viscosity (a higher viscosity) seen by addition of rhamnolipid biosurfactant to the surfactant system at low pH (pH 4 to 5.5).

### Example 2

This example shows the effect of rhamnolipid on the viscosity of a second hand dishwash formulation at low pH.

**Table 4 Hand dishwash formulations**

| **Ingredient** | **B (wt.%)** | **2 (wt.%)** |
|---|---|---|
| Demin water | 86.86 | 86.86 |
| Microcare IT | 0.0009 | 0.0009 |
| Glydant | 0.22 | 0.22 |
| Tetrasodium EDTA | 0.10 | 0.10 |
| R2 Rhamnolipid | - | 1.05 |
| Texapon N701 SLES 1EO | 9.1 | 8.05 |
| Cocoamidopropyl betaine | 1.40 | 1.40 |
| Magnesium sulphate heptahydrate | 2.00 | 2.00 |
| Perfume | 0.32 | 0.32 |
| Colourant | 0.0019 | 0.0019 |

The pH of the base was adjusted with small amounts of diluted citric acid and sodium hydroxide solutions, and the viscosity was measured using the Anton Paar ASC rheometer-using a Bob set-up and reporting the viscosity measured at a shear rate of 23s⁻¹ and shown in tables 5 and 6.

**Table 5 The rheology (viscosity) of the HDW formulation [formulation B (Control)] at a shear rate of 23s⁻¹ when no Rhamnolipid is present**

| **Formulation B (0% Rhamnolipid)** | |
|---|---|
| **pH** | **Viscosity (Cps)** |
| 6.46 | 620 |
| 5.85 | 512 |
| 5.46 | 531 |
| 4.9 | 588 |
| 4.49 | 678 |

**Table 6 The rheology (viscosity) of the HDW formulation [formulation 2 (Invention)] at a shear rate of 23s⁻¹ when 10% of the total active system has been replaced by Rhamnolipid**

| **Formulation 2 (10% Rhamnolipid replacement)** | |
|---|---|
| **pH** | **Viscosity (Cps)** |
| 6.37 | 88 |
| 5.23 | 2080 |
| 4.62 | 3100 |
| 4.20 | 3260 |

This data shows the improvement in viscosity (a higher viscosity) seen by addition of rhamnolipid biosurfactant to the surfactant system at low pH (pH 4 to 5.5).

## Claims

1. Use of a rhamnolipid biosurfactant to increase the viscosity of a fluid cleaning composition at a pH of 4 to 5.5, preferably from 4.5 to 5.5.

2. Use according to claim 1, wherein the rhamnolipid biosurfactant comprises at least 70 wt.% di-rhamnolipid, preferably at least 80 wt.% di-rhamnolipid, preferably of formula: Rha2C₈-₁₂C₈₋₁₂, where the alkyl chains may be saturated or unsaturated.

## Patentansprüche

1. Verwendung eines Rhamnolipid-Biotensids zur Erhöhung der Viskosität einer flüssigen Reinigungszusammensetzung bei einem pH-Wert von 4 bis 5,5, vorzugsweise von 4,5 bis 5,5.

2. Verwendung nach Anspruch 1, wobei das Rhamnolipid-Biotensid mindestens 70 Gew.-% Di-Rhamnolipid umfasst, vorzugsweise mindestens 80 Gew.-% Di-Rhamnolipid, vorzugsweise der Formel: Rha2C₈₋₁₂C₈₋₁₂, wobei die Alkylketten gesättigt oder ungesättigt sein können.

## Revendications

1. Utilisation d'un biotensioactif de rhamnolipide pour augmenter la viscosité d'une composition nettoyante fluide à un pH de 4 à 5,5, de préférence de 4,5 à 5,5.

2. Utilisation selon la revendication 1, dans laquelle le biotensioactif de rhamnolipide comprend au moins 70 % en masse de di-rahmnolipide, de préférence au moins 80 % en masse de di-rahmnolipide, de préférence de formule : Rha2C₈₋₁₂C₈₋₁₂, où les chaînes alkyle peuvent être saturées ou insaturées.
